# EUROPEAN PATENT SPECIFICATION

(11) **EP 2 211 737 B1**
(45) Date of publication and mention of the grant of the patent: **04.06.2014**
(21) Application number: 08799549.4
(22) Date of filing: 15.09.2008
(51) Int. Cl.: A61B 17/70

(54) **SPINAL STABILIZATION SYSTEM WITH TRANSITION MEMBER**
WIRBELSÄULENSTABILISIERUNGSSYSTEM MIT ÜBERGANGSELEMENT
SYSTÈME DE STABILISATION SPINAL AVEC ÉLÉMENT DE TRANSITION

(30) Priority: 20.09.2007 US 858221
(43) Date of publication of application: 04.08.2010
(73) Proprietor: Zimmer GmbH, 8404 Winterthur (CH)
(72) Inventor: ZYLBER, Emmanuel, F-13008 Marseillle (FR); EGLI, Thomas, CH-8604 Kindhausen / Volketswil (CH); THOMPSON, Rosemary, CH-8405 Seen (CH); FROEHLICH, Markus, CH-8362 Balterswil (CH)
(74) Representative: Manitz, Finsterwald & Partner GbR
(86) International application number: PCT/US2008/076387
(87) International publication number: WO 2009/039060

(56) References cited:
- WO-A1-2006/079531
- FR-A- 2 775 583
- US-A1- 2005 149 020
- US-A1- 2005 277 922

## Description

### Field of the Invention

This invention relates to spinal stabilization systems, and more particularly to spinal stabilization systems including a transition member between two fixation elements.

### Background

The spinal column is a highly complex system of bones and connective tissues that provides support for the body and protects the delicate spinal cord. The spinal column includes a series *of* vertebrae stacked one on top of the other, each vertebral body including an inner or central portion of relatively weak cancellous bone and an outer portion of relatively strong cortical bone. The vertebrae in the cervical, thoracic, and lumbar regions of the spine are separated by intervertebral discs, which serve as cushions between adjacent vertebrae to dampen compressive forces experienced by the spine. A vertebral canal containing the spinal cord is formed by the intervertebral foramen of the vertebrae. In spite of the complexities, the spine is a highly flexible structure, capable of a high degree of curvature and twist in nearly every direction. For example, the kinematics of the spine normally includes flexion, extension, rotation, and lateral bending.

There are many types of conditions that can lead to significant pain and affect movement of the spine, including spinal disorders such as scoliosis (abnormal lateral curvature of the spine), kyphosis (abnormal forward curvature of the spine, usually in the thoracic spine), excess lordosis (abnormal backward curvature of the spine, usually in the lumbar spine), and spondylolisthesis (forward displacement of one vertebra over another, usually in a lumbar or cervical spine), as well as conditions caused by abnormalities, disease, or trauma, such as ruptured or slipped discs, degenerative disc disease, fractured vertebra, and the like. In addition to causing pain, these conditions may also threaten the critical elements of the nervous system housed within the spinal canal.

One of the most common methods for treating these conditions is to immobilize a portion of the spine to allow treatment. Traditionally, immobilization has been accomplished by rigid stabilization. For example, in a conventional spinal fusion procedure, a surgeon restores the alignment of the spine or the disc space between vertebrae by installing a rigid fixation rod between pedicle screws secured to adjacent vertebrae. Bone graft is placed between the vertebrae, and the fixation rod cooperates with the screws to immobilize the two vertebrae relative to each other so that the bone graft may fuse with the vertebrae.

Dynamic stabilization has also been used in spinal treatment procedures. Dynamic stabilization does not result in complete immobilization, but instead permits a degree of mobility of the spine while also providing sufficient stabilization to effect treatment. One example of a dynamic stabilization system is the Dynesys® system available from Zimmer Spine, Inc. of Edina, MN. Such dynamic stabilization systems typically include a flexible member positioned between pedicle screws installed in adjacent vertebrae of the spine. A flexible cord can be threaded through a channel in the flexible member and secured to the pedicle screws by a set screw, thereby retaining the flexible member between the pedicle screws while cooperating with the flexible member to permit mobility of the spine.

In some instances, it is desirable to immobilize a portion of the spine using a rigid stabilization system without significantly limiting the mobility or increasing the stress on nearby areas of the spine. Although combining the rigid stabilization system with a dynamic stabilization system would help achieve this objective, there are several challenges associated with doing so. Specifically, there are several challenges associated with combining a flexible element, such as a braided polymer cord, with a rigid element, such as a rigid fixation rod, in a single construct. The cord and rod may be connected or coupled to each other before or during a surgical procedure. But the stiffness of the flexible element is often designed to decrease after placement into a patient's body and as treatment occurs to provide increased range of motion. As a result, there remains a challenge to maintain the rigid element sufficiently coupled to the flexible element after this "relaxation."

Additionally, coupling a rigid rod directly to a flexible cord becomes much more complicated when multiple non-continuous segments of rigid support are desired between certain vertebrae and flexible supports are desired between these rigid support sections. It therefore becomes highly desirable to be able to transition from a rigid element to a flexible element at the pedicle screws secured to the vertebrae.

Document US 2005/0149020 A1 discloses a spinal stabilization system according to the preamble of claim 1.

Document FR 2 775 583 A1 discloses a spinal stabilization system comprising a cord connecting anchor members secured to a patient's spine. The cord is fixed to the anchor members via sleeves though which the cord passes.

Document WO2006079531 A1 discloses a system according to the preamble of claim 1.

It is an object of the present invention to provide a simple yet effective spinal stabilization system which allows to realize complex stabilization patterns.

Said object is achieved by a spinal stabilization system with the features of claim 1.

### Summary

A spinal stabilization system generally comprises an anchor member configured to be secured the patient's spine, a transition member received by the anchor member, and a flexible element coupled to the transition member. The transition member is positioned between a rigid member and a flexible member, which are each positioned adjacent to the flexible element.

In one embodiment, the spinal stabilization system includes first, second, and third anchor members configured to be secured to respective first, second, and third vertebrae within the patient's body. The transition member is received by the second anchor member and includes a body having a first end, a second end, and an axial bore extending between the first and second ends. The flexible element extends through the axial bore in the body of the transition member and between the first and third anchor members, to which the flexible element is secured under tension. The rigid member is configured over the flexible element and positioned between the first and second anchor members. The flexible member is also configured over the flexible element, but is positioned between the second and third anchor members.

### Brief Description of the Drawings

The accompanying drawings, which are incorporated in and constitute a part of this specification, illustrate exemplary embodiments of the invention and, together with a general description of the invention given above, and the detailed description given below, serve to explain the principles of the invention.
Fig. 1 is a partial side elevational view showing a spinal stabilization system according to one embodiment of the invention secured within a patient's body.
Fig. 2 is a cross-sectional view showing a portion of the spinal stabilization system of Fig. 1.

### Detailed Description

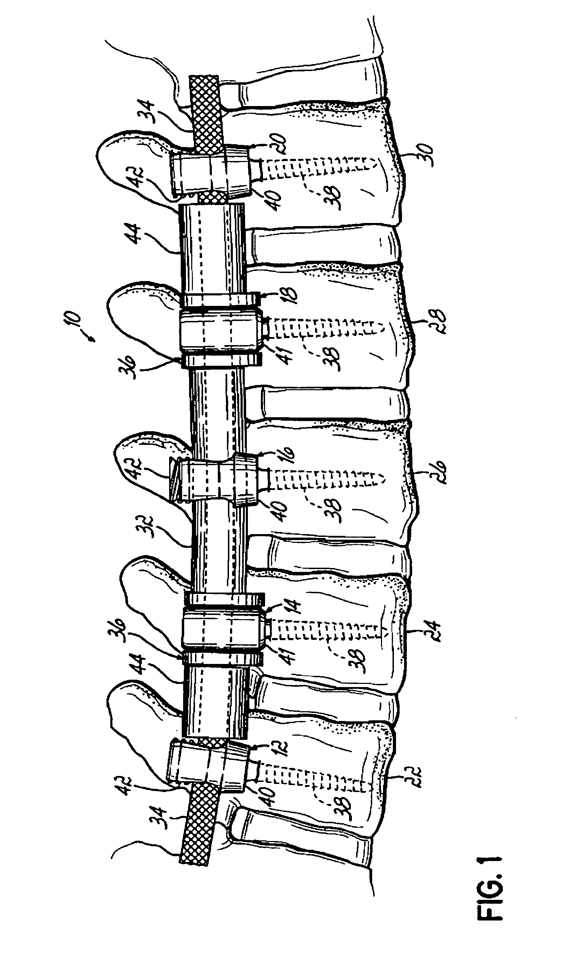

Fig. 1 shows one embodiment of a spinal stabilization system 10 according to the invention within a patient's body. The stabilization system 10 includes first, second, third, fourth, and fifth anchor members 12,14,16,18, 20 secured to respective first, second, third, fourth, and fifth vertebrae 22, 24, 26, 28, 30 within the patient's body. Each of the anchor members 12,14,16,18, 20 may be any type of anchor such as a screw or hook designed to cooperate with a rigid member 32, a flexible element 34, or a transition member 36 to stabilize a portion of the patient's spine. For example, in the embodiment shown in Fig. 1, the first, third, and fifth anchor members 12,16, 20 are pedicle screw assemblies each having a screw body 38, a housing 40 coupled to the screw body 38, and a set screw 42. Each housing 40 receives a flexible element 34 or a rigid member 32, which is then secured to the associated housing 40 by tightening the set screw 42. The second and fourth anchor members 14, 18 in the embodiment shown in Fig. 1 are also pedicle screw assemblies having a screw body 38, but these assemblies have a different housing 41 and a set screw 42 partially hidden in Fig. 1.

As shown in Fig. 1, the flexible element 34 extends from the first *anchor* member 12 to the fifth anchor member 20. The flexible element 34 may be a cord that could be constructed from braided polyethylene-terephalate (PET) fibers or other braided polymer fibers. One or more flexible members 44 are received over the flexible element 34 between the housings 40 to provide additional support during movement of the spine in some embodiments. In one embodiment, the flexible member 44 is a spacer that engages and creates a distance between adjacent housings 40. The flexible members 44 and flexible element 34 cooperate to provide dynamic stabilization of certain portions of the patient's spine. The rigid member 32 is received over the flexible element 34 to provide rigid spinal fixation in certain other portions of the patient's spine. The flexible element 34 may be secured at the first and fifth anchor members 12, 20 so that the flexible element 34 has enough tension to keep the spinal stabilization system 10 properly aligned between the first and fifth anchor members 12,20.

As shown in Fig. *1*, the transition member 36 at the second and fourth anchor members 14,18 provides a transition between the rigid member 32 and one of the flexible members 44. This arrangement enables the spinal stabilization system 10 to combine and alternate between the features of both rigid and dynamic spinal stabilization. The rigid member 32 enables the system 10 to rigidly immobilize a desired area of the spine to promote fusion or other treatment in a specified area, while the flexible member 44 provides additional stabilization without significantly increasing the stress on nearby vertebrae or compromising mobility.

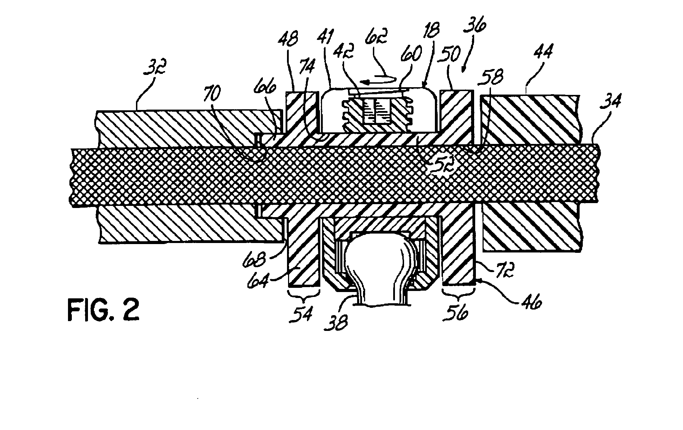

Fig. 2 illustrates the transition member 36 in further detail and how it interacts with the other components of the spinal stabilization system 10. The transition member 36 has a body 46 with an end 48 confronting the rigid member 32 and a second end 50 confronting the flexible member 44. In one embodiment, the body 46 includes a generally cylindrical section 52, a first radial flange 54 coupled to the generally cylindrical section 52 at the first end 48, and a second radial flange 56 coupled to the generally cylindrical section 52 at the second end 50. The cylindrical section 52 is received in a slot 74 of the housing 41 so that first radial flange 54 and second radial flange 56 are positioned on opposite sides of the anchor member 18. An axial bore 58 extends through the body 46 between the first radial flange 54 and the second radial flange 56 to accept the flexible element 34 running through the transition member 36. To secure the transition member 36, the set screw 42 engages internal threads 60 of the housing 41 and is rotated in a direction indicated by arrow 62.

As shown in Fig. 2, the first radial flange 54 may define a generally planar surface 64 with a projection 66 extending outwardly from the first radial flange 54 and received in the rigid member 32. The projection 66 helps position and/or couple the rigid member 32 to the transition member 36. Although Fig. 2 illustrates the projection 66 being an axial flange that defines a portion of the axial bore 58, it will be appreciated that the projection 66 may be any suitable structure for these purposes. Similarly, although a first end 68 of the rigid member 32 is shown as including a counterbore *70* for accommodating the projection 66, other configurations are possible. For example, in a manner not shown herein, the projection 66 may alternatively include a round portion and the first end 68 maybe configured to pivotally couple the rigid member 32 to the projection 66.

The second radial flange 56 may define a generally planar surface 72 configured to interact with the flexible member 44. Either or both of the generally planar surface 64 and generally planar surface 72 may be polished.

While the invention has been illustrated by the description of one or more embodiments thereof, and while the embodiments have been described in considerable detail, they are not intended to restrict or in any way limit the scope of the appended claims to such detail. Additional advantages and modifications will readily appear to those skilled in the art. Therefore, the invention in its broader aspects is not limited to the specific details, representative apparatus, and illustrative examples shown and described.

## Claims

1. A spinal stabilization system, comprising in an assembled state:
an anchor member (12, 14, 15, 18, 20) configured to be secured to the patient's spine; a transition member (36) received by the anchor member; a flexible element (34) coupled to the transition member (36);
a rigid member (32) received over the flexible element (34) to provide rigid spinal fixation in certain portions of the patient's spine;
**characterized in that**
a flexible member (44) received over the flexible element (34);
wherein the flexible member (44) and the flexible element (34) cooperate to provide dynamic stabilization of other portions of the patient's spine;
wherein the transition member (36) is positioned between the rigid member (32) and the flexible member (44);
wherein the transition member (36) comprises a body (46) having a first end (48) confronting the rigid member (32), a second end (50) confronting the flexible member (44), and an axial bore (58) extending between the first and second ends (48, 50), the flexible element (34) extending through the axial bore (58);
**characterised in that**
the body (46) of the transition member (36) further includes a generally cylindrical section (52), a first radial flange (54) coupled to the generally cylindrical section (52) at the first end (48), and a second radial flange (56) coupled to the generally cylindrical section (52) at the second end (50); and **in that**
the first radial flange (54) includes a projection (66) extending outwardly from the first radial flange (54) and configured to be received in the rigid member (32).

2. The spinal stabilization system of claim 1 wherein the anchor member (12, 14, 16, 18, 20) is a pedicle screw assembly configured to be secured to a vertebra (22, 24, 26, 28, 30).

3. The spinal stabilization system of claim 2 wherein the pedicle screw assembly (12, 14, 16, 18, 20) comprises a pedicle screw body (38), a housing (40, 41) having internal threads (60) and a slot (74) configured to receive the transition member (36), and a set screw (42) threadably engaging the internal threads (60) to secure the transition member (36) in the slot (74) of the housing (40, 41).

4. The spinal stabilization system of claim 1 wherein the rigid member (32) and flexible member (44) are each tubular and the flexible element (34) extends through them.

## Patentansprüche

1. Wirbelsäulenstabilisierungssystem, das in einem zusammengebauten Zustand umfasst:
ein Verankerungselement (12, 14, 15, 18, 20), das derart konfiguriert ist, an der Wirbelsäule eines Patienten angebracht zu werden; ein Übergangselement (36), das von dem Verankerungselement aufgenommen ist; ein flexibles Element (34), das mit dem Übergangselement (36) gekoppelt ist;
ein starres Element (32), das über dem flexiblen Element (34) aufgenommen ist, um eine starre Wirbelsäulenfixierung in bestimmten Abschnitten der Wirbelsäule des Patienten bereitzustellen;
**dadurch gekennzeichnet, dass**
ein flexibles Element (44) über dem flexiblen Element (34) aufgenommen ist;
wobei das flexible Element (44) und das flexible Element (34) zusammenwirken, eine dynamische Stabilisierung anderer Abschnitte der Wirbelsäule des Patienten bereitzustellen;
wobei das Übergangselement (36) zwischen dem starren Element (32) und dem flexiblen Element (44) positioniert ist;
wobei das Übergangselement (36) einen Körper (46) umfasst, der ein erstes Ende (48), das dem starren Element (32) gegenüberliegt, ein zweites Ende (50), das dem flexiblen Element (44) gegenüberliegt, sowie eine axiale Bohrung (58) umfasst, die sich zwischen dem ersten und zweiten Ende (48, 50) erstreckt, wobei sich das flexible Element (34) durch die axiale Bohrung (58) erstreckt;
**dadurch gekennzeichnet, dass**:
der Körper (46) des Übergangselements (36) ferner einen allgemein zylindrischen Teil (52), einen ersten radialen Flansch (54), der mit dem allgemein zylindrischen Teil (52) an dem ersten Ende (48) gekoppelt ist, sowie einen zweiten radialen Flansch (56) aufweist, der mit dem allgemein zylindrischen Teil (52) an dem zweiten Ende (50) gekoppelt ist; und dass
der erste radiale Flansch (54) einen Vorsprung (66) aufweist, der sich auswärts von dem ersten radialen Flansch (54) erstreckt und zur Aufnahme in dem starren Element (32) konfiguriert ist.

2. Wirbelsäulenstabilisierungssystem nach Anspruch 1, wobei das Verankerungselement (12, 14, 16, 18, 20) eine Pedikelschraubenbaugruppe ist, die zur Anbringung an einem Wirbel (22, 24, 26, 28, 30) konfiguriert ist.

3. Wirbelsäulenstabilisierungssystem nach Anspruch 2, wobei die Pedikelschraubenbaugruppe (12, 14, 16, 18, 20) einen Pedikelschraubenkörper (38), ein Gehäuse (40, 41) mit einem Innengewinde (60) und einem Schlitz (74), der zur Aufnahme des Übergangselementes (36) konfiguriert ist, sowie eine Einstellschraube (42) umfasst, die mit dem Innengewinde (60) in Gewindeeingriff steht, um das Übergangselement (36) in dem Schlitz (74) des Gehäuses (40, 41) anzubringen.

4. Wirbelsäulenstabilisierungssystem nach Anspruch 1, wobei das starre Element (32) und das flexible Element (44) jeweils rohrförmig sind und sich das flexible Element (34) durch diese erstreckt.

## Revendications

1. Système de stabilisation spinale, comprenant, dans un état assemblé :
un organe d'ancrage (12, 14, 15, 18, 20) configuré pour être fixé sur la colonne vertébrale d'un patient ; un organe de transition (36) reçu par l'organe d'ancrage ; un élément flexible (34) couplé à l'organe de transition (36) ;
un organe rigide (32) reçu par-dessus l'élément flexible (34) pour assurer une fixation spinale rigide dans certaines portions de la colonne vertébrale du patient ;
**caractérisé en ce que**
un organe flexible (44) est reçu par-dessus l'élément flexible (34) ;
dans lequel l'organe flexible (44) et l'élément flexible (34) coopèrent pour assurer une stabilisation dynamique d'autres portions de la colonne vertébrale du patient ;
dans lequel l'organe de transition (36) est positionné entre l'organe rigide (32) et l'organe flexible (44) ;
dans lequel l'organe de transition (36) comprend un corps (46) ayant une première extrémité (48) en face de l'organe rigide (32), une seconde extrémité (50) en face de l'organe flexible (44), et un perçage axial (58) s'étendant entre la première et la seconde extrémité (48, 50), l'élément flexible (34) s'étendant à travers le perçage axial (58) ;
**caractérisé en ce que** le corps (46) de l'organe de transition (36) inclut en outre une section généralement cylindrique (52), une première bride radiale (54) couplée à la section généralement cylindrique (52) à la première extrémité (48), et une seconde bride radiale (56) couplée à la section généralement cylindrique (52) à la seconde extrémité (50) ; et
**en ce que** la première bride radiale (54) inclut une projection (66) s'étendant vers l'extérieur depuis la première bride radiale (54) et configurée pour être reçu dans l'organe rigide (32).

2. Système de stabilisation spinale selon la revendication 1, dans lequel l'organe d'ancrage (12, 14, 16, 18, 20) est un ensemble formant vis pédiculaire configuré pour être fixé sur une vertèbre (22, 24, 26, 28, 30).

3. Système de stabilisation spinale selon la revendication 2, dans lequel l'ensemble formant vis pédiculaire (12, 14, 16, 18, 20) comprend un corps de vis pédiculaire (38), un boîtier (40, 41) ayant un pas de vis interne (60) et une fente (74) configurée pour recevoir l'organe de transition (36), et une vis de blocage (42) qui engage par vissage le pas de vis interne (60) pour fixer l'organe de transition (36) dans la fente (74) du boîtier (40, 41).

4. Système de stabilisation spinale selon la revendication 1, dans lequel l'organe rigide (32) et l'organe flexible (44) sont chacun tubulaire, et l'élément flexible (34) s'étend à travers ceux-ci.
